Europäisches Patentamt

(19) European Patent Office

Office européen des brevets

(11) Numéro de publication: **0 190 964**

**B1**

(12) **FASCICULE DE BREVET EUROPÉEN**

(45) Date de publication du fascicule du brevet:
06.04.88

(51) Int. Cl.⁴: **C 07 C 11/10,** C 07 C 7/163

(21) Numéro de dépôt: **86400136.7**

(22) Date de dépôt: **23.01.86**

(54) **Procédé de production de méthyl-2-butène-2 à partir d'une coupe d'oléfines à 5 atomes de carbone renfermant du méthyl-2-butène-1 et au moins un n-pentène.**

(30) Priorité: **04.02.85 FR 8501612**

(43) Date de publication de la demande:
**13.08.86 Bulletin 86/33**

(45) Mention de la délivrance du brevet:
**06.04.88 Bulletin 88/14**

(84) Etats contractants désignés:
**BE DE GB IT NL SE**

(56) Documents cité:
**EP-A-0 087 658**
**BE-A-735 727**

(73) Titulaire: **INSTITUT FRANCAIS DU PETROLE, 4, Avenue de Bois- Préau, F-92502 Rueil- Malmaison (FR)**

(72) Inventeur: **Boitiaux, Jean- Paul, 4, avenue des Ursulines, F-78300 Poissy (FR)**
Inventeur: **Cosyns, Jean, 50, route d'Herbeville, F-78580 Maule (FR)**
Inventeur: **Derrien, Michel, 5, rue Fillette Nicolas Philibert, F-92500 Rueil- Malmaison (FR)**

# 0 190 964

## Description

Les procédés de cracking tels que le vapocraquage, la viscoréduction, la cokéfaction, le craquage catalytique fournissent des coupes $C_5$ riches en oléfines. Certaines d'entre elles peuvent contenir des proportions importantes de méthyl-butènes (isopentènes). C'est le cas notamment des coupes $C_5$ de craquage catalytique qui peuvent contenir jusqu'à 30 % du mélange de méthyl-2-butène-1, méthyl-2-butène-2, méthyl-3-butène-1. Un exemple de composition d'une telle coupe est donné dans le tableau ci-après :

**Tableau 1**

| HYDROCARBURE | % POIDS | POINT D'EBULLITION °C |
|---|---|---|
| Isopentane | 38,8 | |
| n-pentane | 14,2 | |
| Cyclopentane | 2,1 | |
| Pentène-1 | 1,9 | 29,20 |
| Pentène-2 cis | 6,0 | 37,80 |
| Pentène-2-trans | 8,7 | 36,25 |
| Méthyl-2-butène-1 | 9,5 | 38,60 |
| Méthyl-2-butène-2 | 17,5 | 38,40 |
| Méthyl-3-butène-1 | 1,0 | 20,00 |
| Isoprène | 0,3 | |

On a indiqué dans ce tableau les points d'ébullition des diverses oléfines présentes. On peut voir que ceux-ci sont très proches; en particulier, il est impossible de séparer les méthylbutènes des penténes-2 par distillation fractionnée.

L'invention a pour objet de convertir le méthyl-2-butène-1 d'une coupe $C_5$ d'oléfines, pouvant renfermer des paraffines, en méthyl-2-butène-2 sans hydrogénation substantielle desdits méthylbuténes mais avec hydrogénation substantielle, et de préférence quasi-totale, des n-pentènes.

Lorsque la charge renferme, en outre, une dioléfine $C_5$, l'invention vise à hydrogéner cette dioléfine.

Le méthyl-2-butène-2 est une matière première importante pour de nombreuses synthéses, par exemple pour la production d'alcools ou d'éthers ou pour la production d'isoprène par déshydrogénation. Pour ces applications, la présence d'hydrocarbures saturés en $C_5$ n'est habituellement pas génante.

Lorsqu'on voulait obtenir le méthyl-2-butène-2 dans un bon état de pureté, il était jusqu'à présent nécessaire d'effectuer une extraction à l'acide sulfurique. Celle-ci permet d'obtenir un mélange d'isopentènes à 95-97 % de pureté. Au cours de l'extraction, il se produit un déplacement de la double liaison de sorte que le mélange final contient approximativement 90 % de méthyl-2-butène-2 et 10 de méthyl-2-butène-1. Le procédé à l'acide sulfurique possède cependant plusieurs inconvénients. En premier lieu son coût élevé, dû notamment à la consommation d'acide sulfurique. En effet la présence dans la charge de dioléfines telles que l'isoprène entraîne une surconsommation d'acide sulfurique due à la formation de boues acides qu'il est toujours difficile et coûteux d'éliminer. En deuxième lieu, la coupe isopenténique obtenue n'est pas totalement pure et contient encore une petite quantité d'oléfines linéaires.

On a maintenant trouvé un nouveau procédé qui permet d'obtenir, à partir d'un mélange de pentènes et d'isopentènes, une coupe ne contenant pratiquement plus, comme oléfines, que les isopentènes. Dans cette coupe, la majeure partie des isopentènes est sous forme de méthyl-2-butène-2, les autres hydrocarbures présents étant principalement le n-pentane et l'isopentane. On obtient ainsi les isopentènes avec une pureté proche de 100 % par rapport aux autres oléfines. Les autres hydrocarbures présents, étant paraffiniques, peuvent être considérés comme solvant inerte et ne sont habituellement pas génants dans les procédés de transformation ultérieure du méthyl-2-butène-2.

Le nouveau procédé consiste à faire passer de l'hydrogène et une charge renfermant des oléfines à 5 atomes de carbone, contenant du méthyl-2-butène-1 et au moins un n-pentène, au contact d'un catalyseur supporté comprenant au moins un métal noble du groupe VIII, à une température de 20 à 150°C, par exemple 50 à 150°C et de préférence 60 à 120°C. Selon une caractéristique essentielle du procédé, le mélange des réactifs renferme de 2 à 50 ppm en poids, exprimé en soufre par rapport à la charge d'hydrocarbures, d'au moins un composé du soufre, et la pression est de 5 à 100 bars, par exemple 20 à 100 bars et de préférence 25 à 50 bars.

Il est clair que le procédé concerne essentiellement les charges d'hydrocarbures dont les teneurs respectives en méthyl-2-butène-2 et méthyl-2-butène-1 correspondent à une teneur en méthyl-2-butène-2 inférieure à la proportion d'équilibre thermodynamique, il concerne aussi les charges renfermant du méthyl-2-butène-1 et pas de méthyl-2-butène-2.

Le procédé nouveau consiste ainsi en une hydrogénation sélective des oléfines linéaires en paraffines correspondantes accompagnée d'une isomérisation du méthyl-2-butène-1 en méthyl-2-butène-2. On utilise un catalyseur supporté comprenant au moins un métal noble du groupe VIII. Le palladium est préféré en raison de sa plus grande sélectivité. L'un quelconque des supports usuels peut être utilisé, tel que par exemple l'alumine, la silice ou le carbone. Un catalyseur préféré comprend 0,01 à 2 % (de préférence 0,1 à 0,5 %) en poids de

2

palladium sur support, de préférence sur alumine. Le lit catalytique peut être mobile, mais on préfére un lit fixe. Les conditions opératoires de ce nouveau procédé sont choisies pour réaliser non seulement l'isomérisation du méthyl-2-butène-1 en méthyl-2-butène-2 mais également l'hydrogénation poussée des n-pentènes tout en évitant une hydrogénation substantielle des méthyl-butènes recherchés.

Le débit de la coupe $C_5$ peut être compris entre 0,5 et 20 de préférence entre 1 et 10 litres (à l'état liquide) par litre de catalyseur et par heure. Le débit d'hydrogène est choisi de manière à réaliser l'hydrogénation poussée des n-pentènes sans hydrogéner substantiellement les isopentènes. La quantité en nombre de moles d'hydrogène utilisé sera au moins égale au nombre de moles de n-pentènes de la charge et sera le plus généralement comprise entre 5 et 90 moles pour cent par rapport aux hydrocarbures en $C_5$ introduits.

Le choix judicieux de ces conditions ne suffit cependant pas, en l'absence de composé du soufre, à hydrogéner les pentènes de manière aussi sélective qu'il est souhaitable. Il est donc nécessaire d'opérer en présence d'au moins un composé du soufre, en quantité de 2 à 50 ppm en poids, de préférence 5 à 30 ppm en poids, exprimée en soufre par rapport à la charge d'hydrocarbures. Le composé du soufre peut être naturellement présent dans la charge à traiter ou y être ajouté lorsque cela est nécessaire.

Le soufre peut être présent sous forme de composés inorganiques ou organiques, par exemple sous forme de sulfure d'hydrogène, de thiol, de sulfure, de disulfure, de thiophène, de thiourée, de thioaldéhyde, de thiocétone, de dithiocarbamate, de thiocyanate ou de tout autre composé pouvant comporter ou non des substituants tels que hydroxy, amino, carboxy, halogénure ou analogues. Des exemples sont le diméthylsulfure le diméthyldisulfure, l'éthanethiol, la thioacétone le butanethiol et le sulfure de carbone.

En l'absence de composés sulfurés dans la charge, il n'est pas possible d'éliminer sélectivement les pentènes linéaires; une part importante des isopentènes est hydrogénée en paraffines correspondantes, diminuant ainsi le rendement en méthyl-2-butène-2.

Les exemples suivants, non limitatifs, illustrent l'invention.

**Exemple 1** (comparaison).

Dans cet exemple, on traite une charge d'hydrocarbures ayant la composition suivante:

| | % Poids |
|---|---|
| Pentène-1 | 25 |
| Méthyl-2-butène-1 | 40 |
| Pentane | 35 |

La concentration en soufre dans cette charge est inférieure à 1 ppm en poids.

On fait passer cette charge sur un lit fixe de catalyseur constitué par 0,3 % en poids de palladium déposé sur une alumine gamma tétragonale sous forme de billes et d'une surface de 60 m²/g. Le lit fixe de catalyseur est disposé dans un réacteur tubulaire maintenu dans des conditions sensiblement isothermes. Avant l'emploi, le catalyseur est réduit à pression atmosphérique sous courant d'hydrogène à 100°C pendant deux heures.

Les conditions de traitement de la charge sont les suivantes:

| Pression | : 25 bars |
|---|---|
| Température | : 80°C |
| Débit volumique de charge par volume de catalyseur et par heure | : 5 |
| Débit d'$H_2$ en mole par mole de charge d'hydrocarbures | : 0,3 |

Le produit obtenu dans ces conditions a la composition suivante:

| | % Poids | |
|---|---|---|
| Pentène-1 | 0,1 | |
| Pentènes-2 cis et trans | 2,0 | |
| Méthyl-2-butène-1 | 5,3 | |
| Méthyl-2-butène-2 | 27,6 | 32,9 |
| Pentane | 57,9 | |
| Isopentane | 7,1 | |

On constate que les pentènes n'ont pas été totalement hydrogénés alors qu'une partie appréciable des isopentènes a été transformée en isopentane, la somme de ceux-ci passant de 40 % à 32,9 %.

**Exemple 2** (selon l'invention).

On opère dans les mêmes conditions que celles de l'exemple 1. La charge traitée à la même composition que celle de l'exemple 1 sauf pour la concentration en soufre qui est ici de 6 ppm en poids, sous forme de diméthylsulfure.

La composition du produit obtenu est la suivante:

% Poids

| | | |
|---|---|---|
| Méthyl-2-butène-1 | 7,2 | |
| Méthyl-2-butène-2 | 32,7 | 39,9 |
| Pentane | 60 | |
| Isopentane | 0,1 | |

On constate ici que la totalité des pentènes a été hydrogénée alors que les isopentènes ont été presque totalement conservés. De plus l'isomérisation en méthyl-2-butène-2 a été réalisée, la proportion de cet hydrocarbure correspondant à la proportion prévue par la thermodynamique (82 % du total des isopentènes).

**Exemple 3** (selon l'invention).

Dans cet exemple, on opère avec le même catalyseur que celui des exemples 1 et 2. La charge que l'on traite provient d'un cracking catalytique et sa composition est celle du tableau 1. En outre cette charge contient 12 ppm en poids de soufre sous forme de composés de structure non-déterminée.

Les conditions opératoires sont les suivantes:

| | |
|---|---|
| Pression | : 28 bars |
| Température | : 80°C |
| Débit volumique de charge par volume de catalyseur et par heure | : 3 |
| Débit d'$H_2$ en mole par mole de charge | : 0,3 |

Le produit obtenu a la composition suivante:

| Hydrocarbures | % Poids |
|---|---|
| Isopentane | 39,10 |
| n-pentane | 30,80 |
| Cyclopentane | 2,10 |
| Méthyl-2-butène-1 | 5,04 |
| Méthyl-2-butène-2 | 22,96 |

**Revendications**

1. Procédé de production de méthyl-2-butène-2 à partir d'une charge renfermant des oléfines à 5 atomes de carbone, incluant du méthyl-2-butène-1 et au moins un n-pentène, dans lequel on fait passer un mélange de ladite charge avec de l'hydrogène au contact d'un catalyseur supporté comprenant au moins un métal noble du groupe VIII, à une température de 20 à 150°C, caractérisé en ce que le mélange des réactifs renferme de 2 à 50 ppm en poids, exprimé en soufre par rapport à la charge d'hydrocarbures, d'au moins un composé du soufre et en ce que la pression est de 5 à 100 bars.

2. Procédé selon la revendication 1, caractérisé en ce que le métal noble du catalyseur est le palladium, présent en proportion de 0,01 à 2 % en poids du catalyseur.

3. Procédé selon la revendication 1 ou 2, caractérisé en ce que la température est de 60 à 120°C et la pression de 25 à 50 bars.

4. Procédé selon l'une quelconque des revendications 1 à 3, caractérisé en ce que la teneur en composé du soufre, exprimée en soufre, est de 5 à 30 ppm en poids par rapport à la charge d'hydrocarbures.

5. Procédé selon la revendication 2, caractérisé en ce que le catalyseur est du palladium supporté par de l'alumine.

**Patentansprüche**

1. Verfahren zur Herstellung von 2-Methylbuten-2, ausgehend von einer Charge, die Olefine mit 5 Kohlenstoffatomen enthält einschließlich 2-Methylbuten-1 und wenigstens ein n-Penten, wobei man eine Mischung dieser Charge mit Wasserstoff über einen auf einen Träger aufgebrachten Katalysator, der wenigstens ein Edelmetall der Gruppe VIII enthält, bei einer Temperatur von 20 bis 150°C leitet, dadurch gekennzeichnet, daß die Reaktionsmischung 2 bis 50 Gew.-ppm, berechnet als Schwefel pro Kohlenwasserstoffcharge, wenigstens einer Schwefelverbindung enthält und daß der Druck 5 bis 100 bar beträgt.

2. Verfahren nach Anspruch 1, dadurch gekennzeichnet, daß das Edelmetall des Katalysators Palladium in einem Anteil von 0,01 bis 2 % des Katalysatorgewichts ist.

3. Verfahren nach Anspruch 1 oder 2, dadurch gekennzeichnet, daß die Temperatur 80 bis 120°C und der Druck 25 bis 50 bar beträgt.

4. Verfahren nach einem der Ansprüche 1 bis 3, dadurch gekennzeichnet, daß der Gehalt der Schwefelverbindung, berechnet als Schwefel, 5 bis 30 Gew.-ppm bezogen auf die Kohlenwasserstoffcharge beträgt.

5. Verfahren nach Anspruch 2, dadurch gekennzeichnet, daß der Katalysator Palladium auf einem Aluminiumoxidträger ist.

**Claims**

1. A process for producing 2-methyl-2-butene from a charge containing olefins of 5 carbon atoms, including 2-methyl-1-butene and at least one n-pentene, wherein a mixture of said charge with hydrogen is contacted with a supported catalyst comprising at least one noble metal from group VIII, at a temperature from 20 to 150°C, characterized in that the reactants mixture contains 2 - 50 ppm by weight, expressed as sulfur, with respect to the hydrocarbon charge, of at least one sulfur compound and in that the pressure ranges from 5 to 100 bars.

2. A process according to claim 1, characterized in that palladium is the noble metal of the catalyst and is used in a proportion from 0.01 to 2 % by weight of the catalyst.

3. A process according to claim 1 or 2, characterized in that the temperature is from 60 to 120°C and the pressure from 25 to 50 bars.

4. A process according to any one of claims 1 to 3, characterized in that the sulfur compound content, expressed as sulfur, ranges from 5 to 30 ppm by weight with respect to the hydrocarbons charge.

5. A process according to claim 2, characterized in that the catalyst is palladium supported on alumina.